# EUROPEAN PATENT SPECIFICATION

(11) **EP 2 470 640 B1**
(45) Date of publication and mention of the grant of the patent: **25.02.2015**
(21) Application number: 10744941.5
(22) Date of filing: 23.08.2010
(51) Int. Cl.: B01L 3/00

(54) **MICROFLUIDIC SYSTEM AND METHOD FOR PRODUCING SAME**
MIKROFLUIDISCHES SYSTEM UND VERFAHREN ZU SEINER HERSTELLUNG
SYSTÈME MICROFLUIDIQUE ET SON PROCÉDÉ DE PRODUCTION

(30) Priority: 27.08.2009 DE 102009039956
(43) Date of publication of application: 04.07.2012
(73) Proprietor: NMI NATURWISSENSCHAFTLICHES UND MEDIZINISCHES INSTITUT AN DER UNIVERSITÄT TÜBINGEN, 72770 Reutlingen (DE)
(72) Inventor: SCHUETTE, Julia, 72072 Tübingen (DE); STELZLE, Martin, 72766 Reutlingen (DE)
(74) Representative: Witte, Weller & Partner Patentanwälte mbB
(86) International application number: PCT/EP2010/062246
(87) International publication number: WO 2011/023655

(56) References cited:
- WO-A2-2006/037033
- US-A1- 2007 015 137
- RHEE SEOG WOO ET AL: "Patterned cell culture inside microfluidic devices", 2005, LAB ON A CHIP, VOL. 5, NR. 1, PAGE(S) 102-107, XP002605549, ISSN: 1473-0197 cited in the application the whole document
- WANG L ET AL: "Patterning bio-molecules for cell attachment at single cell levels in PDMS microfluidic chips", MICROELECTRONIC ENGINEERING, ELSEVIER PUBLISHERS BV., AMSTERDAM, NL LNKD- DOI:10.1016/J.MEE.2009.01.030, vol. 86, no. 4-6, 1 April 2009 (2009-04-01), pages 1462-1464, XP026106454, ISSN: 0167-9317 [retrieved on 2009-01-22]
- CHEN-TA HO ET AL: "Rapid heterogeneous liver-call on-chip patterning via the enhanced field-induced dielectrophoresis trap", LAB ON A CHIP, ROYAL SOCIETY OF CHEMISTRY, CAMBRIDGE, GB LNKD- DOI:10.1039/B602036D, vol. 6, 1 January 2006 (2006-01-01), pages 724-734, XP002591870, ISSN: 1473-0197 [retrieved on 2006-05-03]
- DOMINIK G RABUS ET AL: "A Bio-Fluidic-Photonic Platform Based on Deep UV Modification of Polymers", IEEE JOURNAL OF SELECTED TOPICS IN QUANTUM ELECTRONICS, IEEE SERVICE CENTER, PISCATAWAY, NJ, US, vol. 13, no. 2, 1 March 2007 (2007-03-01), pages 214-222, XP011179429, ISSN: 1077-260X, DOI: DOI:10.1109/JSTQE.2007.892073
- WELLE A ET AL: "Photo-chemically patterned polymer surfaces for controlled PC-12 adhesion and neurite guidance", JOURNAL OF NEUROSCIENCE METHODS, ELSEVIER SCIENCE PUBLISHER B.V., AMSTERDAM, NL, vol. 142, no. 2, 30 March 2005 (2005-03-30), pages 243-250, XP004736919, ISSN: 0165-0270, DOI: DOI:10.1016/J.JNEUMETH.2004.08.011
- GOUBKO C A ET AL: "Patterning multiple cell types in co-cultures: A review", MATERIALS SCIENCE AND ENGINEERING C, ELSEVIER SCIENCE S.A, CH LNKD- DOI:10.1016/J.MSEC.2009.02.016, vol. 29, no. 6, 1 August 2009 (2009-08-01) , pages 1855-1868, XP026322570, ISSN: 0928-4931 [retrieved on 2009-03-06]

## Description

The present invention relates to a closed microfluidic system comprising a carrier plate and a cover plate as well as wall regions arranged therebetween, which form a system of channels and/or cavities with an inner surface.

The present invention furthermore relates to a method for producing such closed microfluidic systems.

Such microfluidic systems and methods are widely known from the prior art.

In the context of the present invention, a "covered or closed microfluidic system" is understood to mean a "permanently covered" system, in which carrier plate, cover plate and wall structures provided therebetween are connected to one another permanently and not just temporarily by clipping or clamping.

This differentiates systems according to the invention from those which are customary in laboratory operation, where systems are not stored in large numbers for relatively long periods of time.

In the context of the present invention, "wall regions" are understood to mean microstructures which either are formed directly on the carrier plate and/or cover plate, that is to say are fabricated as constituent parts of carrier plate and/or cover plate during production by micro-milling, micro-injection moulding, hot embossing, etc. However, it is also possible for these microstructures firstly to be fabricated separately from cover plate and carrier plate and only afterwards to be permanently connected to cover plate and carrier plate.

Said wall regions form together with cover plate and carrier plate the channel system and the inner surface thereof. In this case, the surface regions allocated to the wall regions are oriented parallel and/or perpendicularly and/or obliquely with respect to the surface regions of carrier plate and cover plate.

Such microfluidic systems can generally be produced with channels and cavities whose dimensions are comparable to the dimensions of biological cells and tissue structures. Such systems then make it possible to establish and cultivate cells under in vivo-like conditions, that is to say e.g. with the setting of a defined perfusion. It is known that the cells preserve their phenotype under these conditions.

This is relevant to many areas of scientific research and of diagnosis, whether in a research laboratory or in the daily work of a laboratory concerned with routine investigations. This is because in those areas there is a need for complex cell arrangements which are present under as far as possible physiological conditions, that is to say for example in the anatomically correct arrangement of the individual cell types relative to one another, and/or can be perfused physiologically functionally.

One example of the application of such complex cell arrangements is the determination of the toxicity, metabolism and mechanisms of action of medicaments in the pharmaceutical industry. In this case, there is a need for complex, organotypical cell culture systems consisting of "natural" cells which grow in environments which allow differentiation over an appropriately long period of time, and a function comparable to the in vivo situation.

Of particular interest in this connection is, by way of example, an organotypical liver cell coculture with which medicaments are to be tested for toxicity and metabolization. For an organotypical liver cell culture for drug testing it is important here that the hepatocytes are populated or invested at their outer side by endothelial cells, the perfusion of the complex cell culture taking place from the side of the endothelial cells.

Furthermore, there is a need for an organotypical tissue structure such as can be found in the intestine, for example. In this case, too, it is necessary to distinguish between "inside" and "outside" for physiological functional perfusion. The intestinal epithelium consists of a monolayer epithelial layer facing the intestinal lumen, and an underlying layer of mesenchymal cells which maintains the differentiation and function of the epithelial cells. Investigations on the uptake of medicaments on oral administration could be carried out on such a cell assemblage produced in vitro.

A further field of application is the so-called blood-brain barrier, which controls the penetration of substances from the blood into the brain and ensures that the chemical composition of the intracellular fluids of the brain remains substantially constant, which is necessary for precise signal transmission between the nerve cells of the central nervous system. Knowledge about the permeability of the blood-brain barrier for active ingredients and thus their availability in regions of the nervous system is of particular interest in connection with the development of active ingredients.

If such cell cultures are intended to be established in the microfluidic systems mentioned at the outset, one essential task consists in causing the biological cells within the microfluidic systems to adhere to material surfaces in a predetermined structure and with physiological correct surface signals. The interaction with the surface should in this case ideally take place via a coating with so-called extracellular matrix proteins (ECM) in order thus to provide for as far as possible physiological conditions.

Thus, not pre-published DE 10 2008 018 170 of the present applicant describes a microfluidic system that serves for assembling and subsequently cultivating complex cell arrangements. The system comprises a plurality of microchannels via which it can be perfused from outside with a medium. For this purpose, the system is provided with connections for fluidic control.

The microchannels are separated from one another by means of walls in which openings, that is to say cavities, are provided, at which an inhomogeneous electric field can be generated, wherein the structure of the electric field is influenced by webs in the openings and/or microchannels.

By means of the special channel structure and the inhomogeneous electric fields, complex organotypical cell arrangements can be assembled in this microfluidic system at the openings.

The microfluidic system is furthermore provided with different selective coatings in different regions in order to influence the colonization with the cells in a targeted manner. In this case, the colonization can be supported in specific regions by means of an adhesive coating and avoided in other regions by means of a non-adhesive coating. Furthermore, a coating with extracellular matrix proteins (ECM) can be provided in order to support cell growth and cell differentiation. The way in which this functionalization of the individual regions is effected is not described.

In this system it is possible for example to generate an organotypical liver tissue in which hepatocytes and endothelial cells are established in such a way that the hepatocytes are subsequently completely invested by endothelial cells. After this complex structure has been assembled, it is then perfused with nutrient fluid through both microchannels and thus cultivated over prolonged periods of time. If drugs are now added to the medium, they can be tested for toxicity and metabolization. In this case, it is advantageous that the perfusion of the complex cell culture takes place from the side of the endothelial cells, as is the case in intact liver tissue.

The prior art discloses various methods as to how a spatially resolved biofunctionalization of the inner surfaces of microfluidic systems can be realized. This functionalization is carried out here prior to the covering, that is to say closing of the still open system, for which purpose use is made of spotting methods, lithographic methods or micro-contact printing, for example.

A whole-area, complete functionalization of the inner surfaces of a closed system can also be achieved by flushing the system with corresponding solutions with or without prior surface activation with the aid of plasma methods, for example.

Besides use in complex cell cultures of this type, the microfluidic systems mentioned at the outset can also be used for the cultivation of "simple" cell systems which can be used to investigate the behaviour of a wide variety of cells in the broadest sense. For this purpose, too, it is necessary to cultivate the cells in a structured fashion.

The structured cell adhesion required in this connection can be achieved, for example, in accordance with US 2001/0055882 A1, by covering the substrate surfaces with a mask. The surface regions not covered by the mask are then coated with an agent that promotes cell adhesion. In one embodiment, fibronectin, an extracellular matrix protein, is used for coating the non-covered regions. Afterwards, the mask is removed and the non-coated regions are coated with bovine serum albumin (BSA), which is intended to prevent the adhesion of cells there. Afterwards, the biological cells are sown onto these layers, said biological cells settling only on the regions coated with fibronectin.

US 5,470,739 uses a lithographic method to achieve structured protein adhesion and subsequent cell adhesion. In this case, part of the substrate surface is covered with a photoresist, which is partly removed again by photolithography in order to produce a patterned mask that frees regions of the surface. The mask and also the free regions are then coated with collagen. The photoresist is subsequently stripped away, thus resulting in a pattern of collagen-coated regions on the substrate surface. Afterwards, the cells are then sown, which settle on the collagen-coated regions.

Dewez, J.L. et al., "Adhesion of mammalian cells to polymer surfaces: from physical chemistry of surfaces to selective adhesion on defined patterns", in Biomaterials, 1998. 19(16): p. 1441-5, describe a method which involves firstly producing a polystyrene surface with a pattern of strongly and weakly hydrophobic regions. A combined method of photolithography and plasma etching is used for this purpose. The surface structured in this way is then conditioned using a mixture of a surfactant-like block polymer (Pluronic F68^{®}) and an ECM, which has the effect that Pluronic F68^{®} binds to the strongly hydrophobic regions and prevents the binding of the ECM. The ECM binds to the weakly hydrophobic regions and thus allows the selective binding of mammalian cells to the weakly hydrophobic regions.

WO 2006/050617 A1 describes a method in which a cover plate with walls is placed onto a chip and then clamped with the latter in between two pressure plates, such that a temporarily closed microfluidic system composed of channels crossing one another arises, through which system microfluidic flows can be conducted in a targeted manner by means of connections on the pressure plates.

The regions of the chip which correspond to the crossing points of the channels have previously been structured and individually functionalized by means of a lithographic method in such a way that, after being clamped in, they can be activated by means of activating molecules and then colonized with biomolecules or cells of interest. For this purpose, the activating molecules that activate the functionalized region are firstly fed to the crossing via the first channel. The biomolecules or cells that are intended to settle on the activated region are then fed to the crossing via the other channel.

Between the activatable regions, the surface is coated with molecules that prevent the binding of proteins, that is to say "block" these regions.

After the crossing points have firstly been activated and then colonized with biomolecules or cells, the chip is intended to be used, in particular, for investigating proteins or cells which have been established on the activated regions.

The known method therefore serves to colonize a chip with proteins or cells in a structured manner. The planar microarrays thus produced are then used to carry out immunoassays, which are then read out by means of fluorescence measurements or the like.

A permanently closed microfluidic system within the meaning of the present invention is therefore not actually disclosed in WO 2006/050617 A1; rather, an only temporarily closed system is produced which is merely used for the structured colonization of a chip. The chip is subsequently removed again for the actual experiments.

Functionalized and non-functionalized poly(L-lysine)-g-polyethylene glycols (PLL-g-PEG) are mentioned as sole embodiment for the functionalization and blocking of the corresponding regions of the chip.

This method is very complex since it requires the separate coating of activatable and non-activatable regions of the chips.

Furthermore, the known method only allows the production of planar, two-dimensional functionalizations and is not suitable for the spatially resolved functionalization of arbitrarily shaped three-dimensional regions in microfluidic structures.

EP 2 014 763 A1 discloses a microfluidic container having concave and convex structures in which cells are established, which are supplied with nutrients via microfluidic supply lines. The convex channels can be coated with a cell adhesion promoter.

Rhee, S.W. et al., "Patterned cell culture inside microfluidic devices", in Lab Chip, 2005, 5(1): pp. 102-7 describe a method in which a complete substrate is coated with poly-L-lysine, a patterned stamp is applied and the non-covered surface is freed of the protein by plasma etching. After the stamp has been stripped away, a microfluidic channel system is adhesively bonded at the surface regions freed of protein. In the microfluidic system thus formed, neurons were then selectively applied to the PLL-coated surface and cultivated in the system.

A method for the spatially resolved, microstructured biofunctionalization of arbitrary, in particular including three-dimensionally shaped regions in closed microfluidic systems which make it possible to assemble complex, three-dimensional structures and can be produced by the customary mass production methods for covered microfluidic systems is not known from the prior art discussed so far.

This is due to the fact that the conventional covering methods for polymeric microfluidic systems, such as laser welding, adhesive bonding, lamination and others, are not compatible with biomolecules of any type since they would lead to the disruption thereof.

For this reason, the ligands such as biotin, NTA, single-stranded DNA and antibodies as proposed in WO 2006/050617 A1 for the subsequent binding of the activation molecules are also not suitable for a functionalization of the respective regions at least when the microfluidic systems are intended to be produced in large numbers in an efficient and cost-effective manner.

Patrito et al., "Spatially Controlled Cell Adhesion via Micorpatterned Surface Modification of poly(dimethylsiloxane)", in Langmuir. 2007 Jan. 16; 23(2):715-9, disclose a method for the surface modification of PDMS to promote localized cell adhesion and proliferation. In this method, thin metal films are deposited onto PDMS through a physical mask in the presence of a gaseous plasma, leading to topographical and chemical modifications of the polymer surface.

Hook et al., "Patterned and Switchable surface for Biomolecular Manipulation", in Acta Biomaterials 5 (2009) 2350 - 2370, disclose a microfluidic film with a pattern of spatially activated regions for cell binding. This film is produced using a PDMS mold containing grooves which form microchannels when put onto the surface of a substrate, in the disclosed case a film of PLA-PEG block copolymer modified with biotin. Flowing avidin through the microchannels produces spatially activated regions on the surface. Then, biotinylated peptides are flowed through the microchannels to produce a surface for cell binding. After the mould has been removed, cells are seed on the surface, whereby the cells only bind to the activated surface areas that formed the bottom of the temporarily available microchannels.

US 2007/00151179 A1 discloses a microfluidic chip for isolation of nucleic acids from biological samples. Such chip is provided with surface-modified channels packed with polymer-embedded particles. Using photoinitiated grafting, patterns with different surface properties are created and form a solid phase extraction column within the channels. To perform immunoassays, a Protein A layer is immobilized in unstructured fashion on the whole the surface of the channels.

WO 2006/037033 A1 discloses a method wherein a substrate is pre-functionalized by using O₂ plasma. Such substrate is bonded with a PDMS channel and thereafter backfilled with a fragile ECM protein after assembling the substrate with the microfluidic PDMS piece.

Wang et al., Microelec. Eng., Vol. 86, No. 4-6, April 2009, pages 1462-1464 discloses a microfluidic device wherein PLL is structured by photolithography. The open system is completely fabricated and thereafter covered with a protective layer that can be removed by rinsing the closed system. The inner surfaces of the system are further passivated by rinsing with PLL-PEG.

Ho et al., Lab on a chip, 2006, Vol. 6, No. 6, pages 724-734 discloses a microfluidic chip whereon a planar structure of liver cells can be established in a 2D arrangement. An inhomogeneous electric field with defined gradients is generated through the geometric structure and arrangement of the electrodes and brings together cells of two cell types which are randomly present in a chamber to give a desired planar tissue pattern.

US 2007/015137 A1 discloses a microfluidic device with an observation chamber, said device comprising a bottom plate and a cover plate irreversibly bound to the bottom plate.

In view of the above, it is an object of the present invention to provide systems and methods of the type mentioned at the outset that are compatible with the customary mass production methods, in particular covering methods for microfluidic systems, and nevertheless allow a spatially resolved, three-dimensional biofunctionalization.

This object is achieved according to the invention by means of a microfluidic system of the type mentioned at the outset in which selected regions of the inner surface are selectively functionalized by acid groups formed in the functionalized regions on account of selective irradiation with short wave-length light such that the functionalized regions are hydrophilized.

Especially, arbitrarily selected regions of the inner surface are selectively functionalized such as to enable subsequent activation of the functionalized regions for the binding of biological cells and/or of bio-molecules.

This allows activation of the functionalized regions even after long-time storage of the closed microfluidic system and subsequent formation of complex, three-dimensional structures of biomolecules.

Furthermore, the object underlying the invention is achieved by means of a method for producing the novel closed microfluidic system, comprising the steps:
a) providing a carrier plate and a cover plate, wall regions being provided on the carrier plate and/or the cover plate,
b) selectively functionalizing selected regions of the inner surface on the carrier plate, the cover plate and/or the wall regions, in that the selective regions are selectively irradiated with short wave-length light in order to form acid groups in the surface of the selected regions, such that the selected regions are hydrophilized, and
c) permanently connecting carrier plate, cover plate and wall regions to form the closed microfluidic system.

Finally, the present invention also relates to a method for the spatially resolved colonization of the novel closed microfluidic system with biological cells, comprising the steps:
- providing the novel microfluidic system,
- rinsing the microfluidic system with at least one activation solution in order to activate the functionalized regions for the binding of the cells,
- rinsing the microfluidic system with a cell solution in order to bind the cells to the activated regions.

The present invention also relates to a method for establishing a closed microfluidic flow system in which substances contained in a reaction solution come into contact with differently activated regions, comprising the steps:
- providing the novel microfluidic system, and
- rinsing the microfluidic system with at least one activation solution in order to activate the functionalized regions for a reaction with the substances.

The object underlying the invention is completely achieved in this way.

Namely, the inventors of the present invention have realized that it is possible to functionalize arbitrary regions both of the carrier plate and of the cover plate and of the wall regions by irradiation with short-wavelength light, such that complex cell structures can then be assembled in the closed system, as is described for example in not pre-published DE 10 2008 018 170.

In this case, it is in particular advantageous, that surface regions which do not lie parallel to the plane of cover plate and/or carrier plate can also be functionalized in this way, which allows the subsequent formation of complex, that is to say three-dimensional, cell structures.

It is furthermore advantageous, that the functionalization can be achieved rapidly and simply if the properties of the material surfaces of carrier plate, cover plate and wall regions are utilized in order to provide the non-functionalized regions without further work steps; only the regions to be activated then have to be irradiated. This holds true, in particular, for polymeric materials in which the UV irradiation effects the formation of acid groups. Other conventional materials for fluidic microsystems, such as glass, silicon or silicon nitride, have to be made non-adhesive beforehand, which can be achieved e.g. by silanization with a hydrophobic silane.

Furthermore, it is advantageous that the chosen functionalization allows a long storage time for the novel systems. The acid groups generated by the irradiation can already per se be stably stored for several months. By filling the permanently closed system with corresponding gases or liquids and subsequent welding-in it is possible, however, to ensure an even considerably longer storage time for the systems packaged in a sterile fashion. In this way it is possible to prevent alcohol molecules from interacting with the acid groups and forming esters.

It is known, moreover, that, by means of irradiation with short-wavelength UV light (< 200 nm) planar plastic surfaces are hydrophilized by acid groups being formed in the irradiated surfaces. As a result, the surface thus functionalized becomes accessible to protein binding. This has already been shown in various publications for different polymers such as e.g. polystyrene, poly(methyl)methacrylate, polycarbonate or cyclic olefin copolymers.

Welle, A., et al., "Photo-chemically patterned polymer surfaces for controlled PC-12 adhesion and neurite guidance", in J Neurosci Methods, 2005, 142(2): pp. 243-50 and Holländer et al., "Structured R2R Functionalisation of Polymer Film Surfaces by a Xenon Excimer Lamp", in Plasma Process. Polym., 2007. 4: p. 5, were able to show that, by means of the UV treatment, the surface of different polymers is hydrophilized and carboxylic acid groups are formed which remain stable for many months. They report on directed growth of neurites and liver carcinoma cells after selective UV irradiation and incubation with a BSA/Pluronic® mixture.

Rabus, D.G., et al., "A Bio-Fluidic-Photonic Platform Based on Deep UV Modification of Polymers", in Selected Topics in Quantum Electronics, IEEE Journal of 2007, 13(2): pp. 214-222, were able to adhere fibroblasts on UV-activated regions which were incubated with a mixture of laminin and Pluronic®.

EP 2 O11 629 A1 discloses an open microfluidic system fabricated on the surface of a polymeric foil or carrier. A capillary channel is punched out at said surface and subsequently the surface is morphologically and/or chemically modified by spatially resolved irradiation with laser light. Thereby, a pattern of hydrophilic and hydrophobic areas is provided to selectively modify the wettability by a fluid sample.

However, all the aforementioned publications concerning the hydrophilization of plastic surfaces by irradiation using short-wave UV light were implemented in culture dishes or static well systems which do not allow perfusion or assembly of complex, three-dimensional structures, as is now possible when the novel microfluidic systems are used as intended.

The invention for the first time affords the possibility for the spatially resolved, microstructured biofunctionalization or passivation of closed microfluidic systems with arbitrary, including sensitive, biomolecules or molecules that have a passivating effect. The invention provides corresponding systems for this purpose which, by means of mere flushing of the fully completed, closed (covered) system, are biofunctionalized at arbitrary predefined regions of the inner surfaces by means of the binding of the biomolecules, or are passivated, if appropriate, on the remaining surface regions by means of the binding e.g. of PEG derivatives.

In this case, the problem of the incompatibility of the sensitive biomolecules with the customary covering methods is solved by the fact that, only after the covered microsystem has been fully completed, the biomolecules are introduced by means of a flushing process. The spatially resolved binding of these molecules is achieved by means of the structured UV activation of the system prior to covering. This UV activation results in the formation of acid groups and withstands both the covering process and relatively long storage times - up to 4 months or more - which is crucial for an application. Microsystems chemically activated in this way are then biofunctionalized only directly before use on the part of the user.

The spatially resolved adhesion of protein is achieved by a stable chemical activation of the polymer surface by means of UV irradiation (e.g. through a shadow mask). Acid groups form in the irradiated surface regions in the process, which enter into a covalent bond with amino groups of the polymers. This UV activation takes place on the open microfluidic system, that is to say before the covering process, by which the activation is not influenced, however.

The invention, therefore, for the first time provides a closed microfluidic system which on its inner surface comprises arbitrarily distributed functionalized regions which, even after relatively long storage of the system, can firstly be activated by the binding of ligands and then be colonized with biological cells.

In the case of the new production method, in step b), the selected regions are selectively irradiated with short-wavelength light in order to form acid groups in the surface of the selected regions.

In this case, it is preferred if, in step b), the selected regions are irradiated with short-wavelength light through a shadow mask or via a scanning laser system, the wavelength of said light being in the range of 150 to 220 nm, preferably 180 to 200 nm, more preferably approximately 185 nm. This method also allows the functionalization of wall areas which are not oriented parallel to the carrier plate or cover plate.

In the case of the novel method for the spatially resolved colonization of the novel microfluidic system with biomolecules such as, for example, proteins, especially ECM, enzymes, or scavenger molecules, it is consequently preferred if the activation solution contains passivation molecules which adhere to the non-functionalized regions and lead to the passivation thereof, wherein the activation solution preferably contains polyethylene derivatives, preferably a block copolymer with polyethylene glycol chains.

The activation solution preferably contains ligands which adhere to the functionalized regions and lead to the activation thereof, that is to say promote the adhesion of biological cells to the selected regions of the inner surface. The ligands are preferably protein molecules, preferably extracellular matrix proteins.

The coating with the sensitive ECM molecules is therefore effected only after full completion of production including covering, namely by flushing or rinsing the microsystem for example with a Pluronic® / protein solution. Proteins bind on the irradiated areas. The previously unirradiated and therefore hydrophobic regions are passivated by the adhesion of Pluronic®, a block copolymer with polyethylene glycol chains, that is to say that neither proteins nor cells adhere there.

In this case, the novel microfluidic systems are suitable for arbitrary proteins since they lead to binding via amino groups.

The novel method for establishing a microfluidic flow system accordingly makes it possible for the microfluidic systems according to the invention to be selectively activated at their functionalized regions by means of flushing with activation solutions only on the part of the user, that is to say even after relatively long transport and/or storage times. The reaction solution is then directed through the system, such that the substances contained therein can be converted in a reaction cascade, for example. The reaction solution that leaves the system can subsequently be analysed.

In this case, the activation solution preferably contains functional molecules which adhere to the functionalized regions and lead to the activation thereof, wherein the functional molecules can comprise enzymes and/or scavenger molecules.

If enzymes are used as functional molecules, the substrate molecules contained in the reaction mixture can then be converted, if appropriate, in successive stages. By contrast, if scavenger molecules such as antibodies or aptamers are used as functional molecules, then the microfluidic system can also be used for diagnosis purposes by the analysis of the selective binding of ligands contained in the reaction mixture.

A customer-specific biofunctionalization by the user thus becomes possible, in which case a very simple process is available with the flushing, in contrast to otherwise customary methods such as spotting, lithography, micro-contact printing.

The method can be employed even with very sensitive biomolecules since the latter do not have to withstand a covering process or a storage time.

The novel microfluidic system is preferably provided with connectors for fluidic control.

It is advantageous here that microfluidic flows can be directed through individual channels and channel regions in a targeted and controlled manner in order to allow selective activation and colonization of individual functionalized regions.

It is preferred in this connection if at least one pressure barrier is provided in the channels, which is preferably formed by at least one cross-sectional reduction in the channel system, wherein the channel system furthermore preferably has at least one longitudinal channel which is connected to an inlet and from which proceed at least two transverse channels which are each connected to a dedicated outlet, wherein, with further preference, a pressure barrier is provided in the longitudinal channel upstream of each branching transverse channel.

Here, too, it is in each case advantageous that microfluidic flows can be controlled in a rapid and simple manner. By way of example, targeted microfluidic flows can be constrained which enable the individual functionalized regions to be colonized with different biomolecules.

Generally, it is also preferred if the carrier plate, the cover plate and/or the wall regions comprise a polymeric material, which is preferably selected from the group comprising PDMS (polydimethylsiloxane), PMMA (poly(methyl methacrylate)), polystyrene, PEEK (polyether ether ketone), and COC (cyclic olefin copolymer).

Polymers such as, for example, PDMS (polydimethylsiloxane), PMMA (poly(methyl methacrylate)), polystyrene, PEEK and COC (cyclic olefin copolymer), have proved to be suitable material for the microstructure since they can be functionalized directly by UV irradiation. Furthermore, it is advantageous that the unirradiated regions are hydrophobic and can be passivated for example by flushing with surfactant-like block polymers such as Pluronic®.

It is furthermore preferred in this connection if the carrier plate, the cover plate and/or the wall regions comprise a material selected from the group comprising glass, silicon or silicon nitride, and which is preferably provided with a hydrophobic coating.

After prior suitable coating it is also possible to use glass or silicon, if appropriate coated with an insulating layer composed e.g. of silicon oxide or silicon nitride. For this purpose, these materials can be pretreated with a monolayer of silane derivatives, which can then, by means of UV irradiation, be made reactive, that is to say hydrophilic, or become inactive; in this respect, see for example Dulcey et al., "Deep UV Photochemistry of Chemisorbed Monolayers: Patterned Coplanar Molecule Assemblies", in Science, 1991, Vol. 252, 551-554, or Calver, "Lithographic Patterning of Self-Assembled Films", in J. Vas. Sci. Technol. B 11(6), 1993, 2155 - 2163.

Transparent, non-conductive materials are preferably used, although the above enumeration should be understood only as by way of example.

The microstructure can be produced by means of suitable methods known per se for microstructuring such as, for example, photolithography in combination with plasma etching methods or wet-chemical etching methods and, in the case of polymer materials, by micro-injection moulding or hot embossing.

It is furthermore preferred if an electrode arrangement is provided in the channel system.

In this case, it is advantageous that homogeneous or inhomogeneous fields can be generated in the channel system, by means of which fields the assembly of biological cells can be controlled, as is described in not pre-published DE 10 2008 018 170 in the name of the present applicant, mentioned at the outset.

Finally, it is preferred if the microfluidic system is filled with a fluid that prevents an interaction of the functionalized regions with alcohol molecules, and if it is packaged in a sterile fashion.

In this way, the storage life of the novel microfluidic system can be significantly lengthened again.

Further advantages are evident from the description and the accompanying drawing.

It goes without saying that the features mentioned above and those yet to be explained below can be used not only in the combination respectively specified, but also in other combinations or by themselves, without departing from the scope of the present invention.

Embodiments of the invention are illustrated in the drawing and are explained in greater detail in the description below, where
- Figure 1: shows a schematic plan view in the form of a detail not true to scale of a carrier plate of the novel microfluidic system along the line I-I from Figure 2;
- Figure 2: shows a schematic sectional illustration not true to scale through the microfluidic system from Figure 1 along the line II-II therein;
- Figure 3: shows in an illustration like Figure 2 a perspective view in the form of a detail of a further embodiment of the novel microfluidic system, wherein channel electrodes are provided on the channel base and cover;

- Figure 4: shows in an illustration like Figure 3 a further embodiment of the novel microfluidic system, wherein channel electrodes are provided on the side walls;
- Figure 5: shows a basic illustration of how selected regions of a material surface are selectively functionalized and subsequently activated;
- Figure 6: shows a basic illustration of how selected regions of a material surface are selectively activated and subsequently colonized with biological cells;
- Figure 7: shows a plan view of a further embodiment of the novel microfluidic system, in which the cover plate has been removed;
- Figure 8: shows a longitudinal section in the form of a detail through the microfluidic system from Figure 7 along the line VIII-VIII therein; and
- Figure 9: shows the microfluidic system from Figure 7, wherein valves have been connected to the connectors for microfluidic control with the aid of which valves the system can be flushed and selected regions can be selectively activated and colonized with different biological cells.

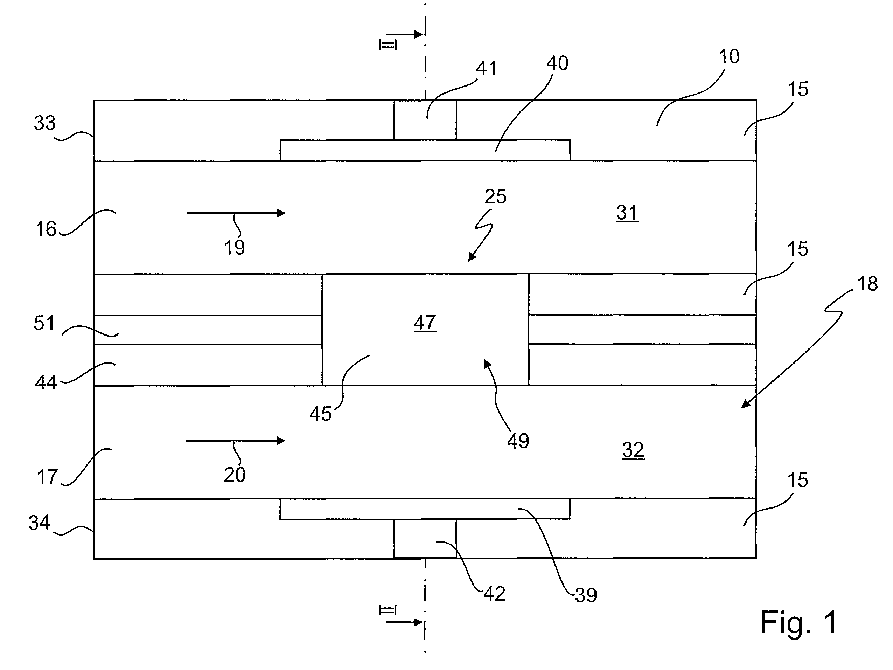

Figure 1 illustrates a schematic plan view in the form of a detail not true to scale of a carrier plate 10 of a first embodiment of the novel microfluidic system 12.

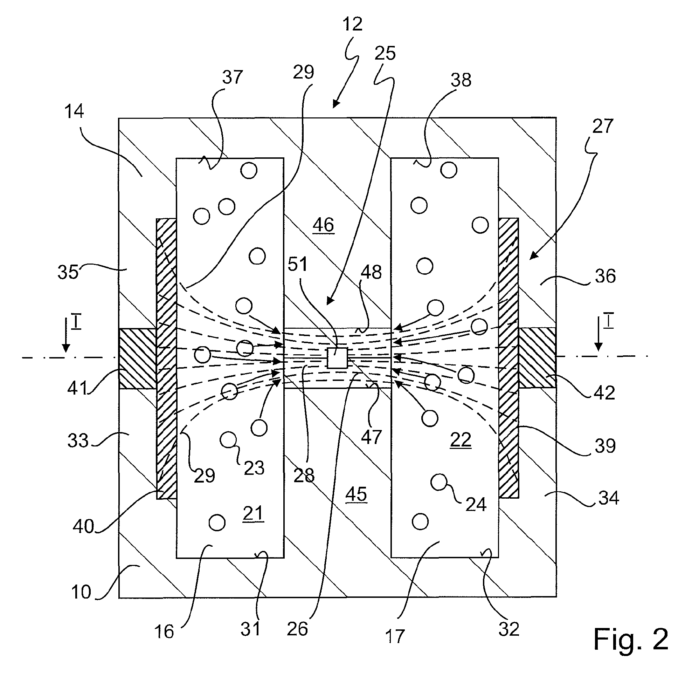

Figure 2 shows a section transversely through the entire microfluidic system 12 along the line II-II from Figure 1, while the plan view in Figure 1 is viewed along the line I-I from Figure 2.

The microfluidic system 12 has a cover plate 14, which corresponds to the carrier plate 10 in terms of the geometrical construction and which closes the carrier plate 10.

Various wall regions 15 can be seen on the carrier plate 10 and the cover plate 14, said wall regions here being formed integrally with the carrier plate 10 and the cover plate 14, respectively.

Two microchannel segments 16, 17 run through the microfluidic system 11 - in a manner delimited laterally by the wall regions 15 - parallel to and at a distance from one another, and are formed partly in the carrier plate 10 and partly in the cover plate 14 in the example shown. It goes without saying that the microchannel segments 16 and 17 can also be formed entirely in the carrier plate 10 or in the cover plate 14, and the cover plate 14 and the carrier plate 10 then merely form a channel cover and channel base, respectively.

Carrier plate 10, cover plate 14 and wall regions 15 are permanently connected to one another by laser welding or adhesive bonding, for example, and thus form a closed microfluidic system 12 with a channel system 18, which is provided with an inner surface formed by the corresponding surface regions of carrier plate 10, cover plate 14 and wall regions 15.

Via the microchannel segments 16, 17, the microfluidic system 12 is perfused from outside in directions of flow 19 and 20 defined by the microchannel segments 16, 17, with medium indicated at 21 and 22 in Figure 2. With the medium 21, 22, nutrients and test substances can be supplied, and metabolitic products can be removed. Furthermore, cells 23, 24 can be transported in the medium 21, 22, which cells assemble to a complex cell arrangement.

The microchannel segments 16, 17 are separated from one another by a wall structure 25, in which an opening 26 connecting the two microchannel segments 16, 17 to one another, that is to say a cavity, is provided.

Furthermore, an electrode arrangement 27 is provided in the microfluidic system 12, by means of which electrode arrangement an inhomogeneous electric field 28 is generated in the region of the opening 26, some field lines 29 of which field are illustrated in a dashed manner by way of example in Figure 2.

Said field 28 moves the cells 23, 24 towards the opening 26, where they assemble and form a complex cell arrangement (not shown in Figure 2). In this case, use is made of the effect of field-induced dielectrophoresis.

It is evident in Figures 1 and 2 that the carrier plate 10 has outer walls 33, 34 which extend upwards from the respective channel base 31, 32 and correspond to outer walls 35, 36 on the cover plate 14 which extend from the respective channel cover 37 and 38. The outer walls 33, 34, 35, 36 bear on one another with their end surfaces facing towards one another.

The outer walls 33, 34, 35, 36 and the wall region 25 form the abovementioned wall regions 15, while the channel bases 31, 32, the channel covers 37, 38, and the surface regions of the outer walls 33, 34, 35, 36, of the wall region 25 and of the opening 26 form the inner surface of the channel system 18.

Channel electrodes 39 and 40 of the electrode arrangement 27 are arranged in or on the outer walls 33, 34, 35, 36 opposite the opening 26, and can be connected via leads 41 and 42, respectively, to an electrical AC voltage generator 43 (not visible in Figure 2) having a variable frequency f and variable voltage swing Upp.

The wall structure 25 comprises a partition 44, which is formed by corresponding regions of cover plate 14 and carrier plate 10 which, like the outer walls 33, 34, 35, 36, bear on one another. In the region of the opening 26, the partition 44 is formed with webs 45, 46 which are set back relative to the bearing area and whose end surfaces 47 and 48, respectively, face one another and delimit the opening 26 between them.

The webs 45, 46 run in the direction of flow 19, 20, such that the opening 25 has the form of an elongated gap 49.

A further microchannel 51 runs in the partition 44 parallel to and between the microchannel segments 16, 17 and is fluidically connected to the gap 49, such that material can be removed from the region of the gap 49.

In this case - as shown in Figure 1 - the further microchannel 51 can pass through the gap 49, that is to say be connected on both sides respectively to the gap 49 and the opening 26, but it can also be provided only on one side of the gap 49, which is advantageous in particular for investigating organotypical liver structures when the further microchannel 51 serves as bile duct.

The microfluidic system 12 is fabricated from a dielectric material, such that the field structure is concomitantly determined by the geometry described in this respect. The field 28 has its highest field density in the region of the gap 49, the shape of the field substantially being determined by said geometry, and the field strength by the voltage swing Upp.

Polymers such as, for example, PMMA, polystyrene, PEEK, COC (cyclic olefin copolymer) have proved to be suitable material for the microfluidic system 12. Transparent, non-conductive materials are preferably used, wherein the above enumeration should be understood only as by way of example.

The microfluidic system 12 can be produced by means of suitable methods known per se for microstructuring such as, for example, photolithography in combination with plasma etching methods or wet-chemical etching methods and, in the case of polymer materials, by micro-injection moulding or hot embossing.

As already mentioned, the microfluidic system 12 is suitable e.g. for establishing an organotypical liver structure in which a liver sinusoid having two rows each of approximately 20 to 30 hepatocytes in succession is intended to be assembled in the gap 49.

In this case, the microfluidic system 12 is provided with different selective coatings in different regions. In this case, colonization is supported in the region of the gap 49 by means of an adhesive coating and avoided in the microchannel segments 16, 17 by means of a non-adhesive coating.

According to the invention, the surface regions of the gap 49 are hydrophilized by selective formation of acid groups, while the remaining regions of the inner surfaces are hydrophobic, that is to say have the property of the untreated material of which the microfluidic system 12 consists.

By means of a coating with extracellular matrix protein, the hydrophilized surface regions of the gap 49 are activated for cell adhesion, wherein cell growth and cell differentiation are simultaneously supported thereby.

The remaining, hydrophobic surface regions, which were not functionalized, are coated with Pluronic™, a block copolymer with polyethylene glycol chains, which leads to a passivation of these surface regions, such that no cells can adhere there.

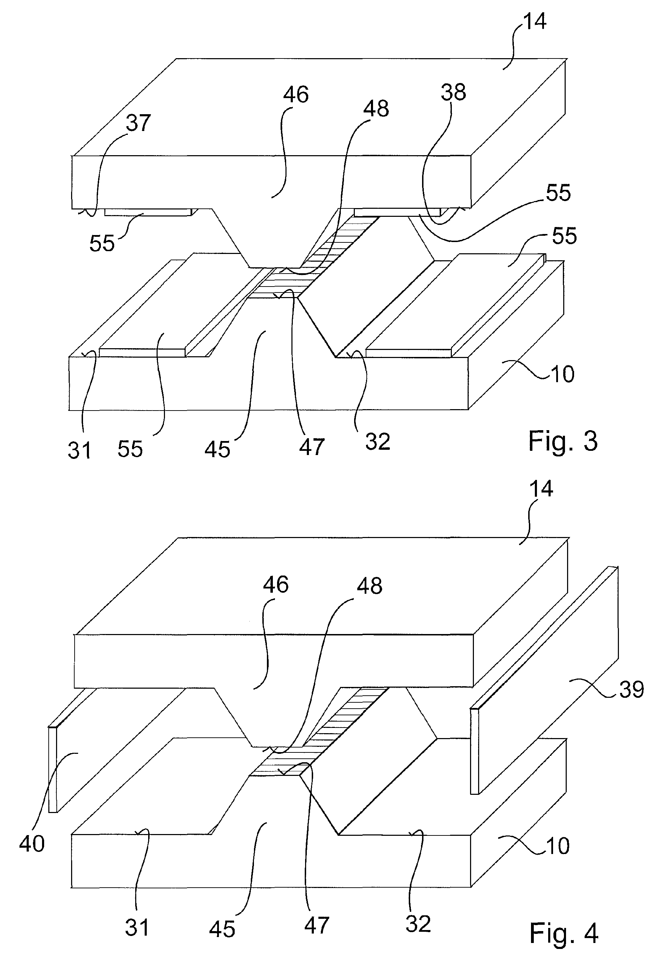

While the webs 45 and 46 are rectangular in cross section in the embodiment in Figures 1 and 2, the webs can also be formed in a trapezium-shaped manner, as is shown in Figures 3 and 4. By means of the trapezium-shaped web structure, the inhomogeneous electric field can be influenced further, such that a field structure arises which is particularly suitable for assembling cells.

While in Figure 4 the channel electrodes 39, 40 are arranged as in Figures 1 and 2 on the outer walls (not shown in Figure 4), in the embodiment in accordance with Figure 3 channel electrodes 55 are arranged on the channel base 31, 32 and on the channel cover 35, 38.

It goes without saying that it is also possible to provide channel electrodes both on the outer walls and on the channel base and channel cover.

The inhomogeneous field that forms can be influenced further by the chosen arrangement of the channel electrodes 39, 40, 55.

The surfaces 47, 48 of the webs 45, 46 are hydrophilized according to the invention such that they can be activated with ECM, while the remaining surfaces are hydrophobic.

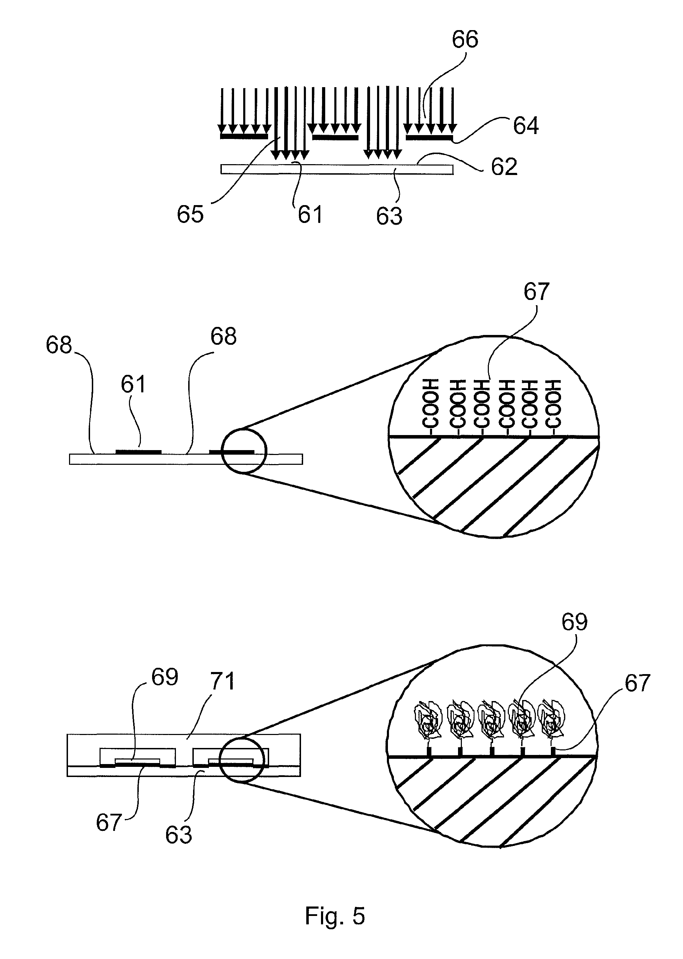

Figure 5 illustrates, in principle, how selected regions 61 of a surface 62 of a substrate 63 can be functionalized.

For this purpose, a shadow mask 64 is arranged above the surface 62, in which shadow mask are provided perforations 65 corresponding to the regions 61 to be functionalized on the surface 62.

In this case, the substrate 63 is a customary polymer such as is used for producing microfluidic systems. Alternatively, the substrate can also consist of glass or silicon, in which case it must then have been provided with a hydrophobic coating beforehand.

Through the shadow mask 64, the substrate 63 is then irradiated for a time duration of 25 min, for example, with a short-wavelength light 66, which has a wavelength of 185 nm in the present case.

As a result of this irradiation, the selected regions 61 are hydrophilized and COOH acid groups 67 are formed, which is indicated on the right in the centre of Figure 5.

This formation of acid groups 67 in surfaces of polymeric materials is already known, in principle, from the publications mentioned at the outset.

The formation of the acid groups 67 in the selected regions 61 functionalized the latter, that is to say hydrophilized in the present case, hydrophobic regions 68 remaining between the regions 61 thus functionalized, as is shown in the middle illustration in Figure 5.

The hydrophilized regions 61 can then be activated by flushing with a protein solution for the adhesion of biological cells, protein 69 binding to the acid groups 67. By contrast, the hydrophobic regions 68 are passivated by flushing with Pluronic™.

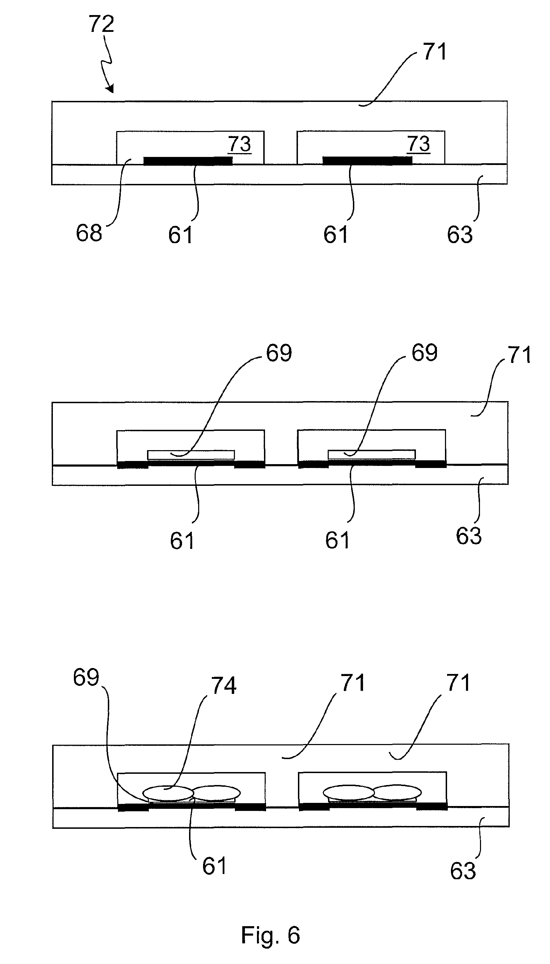

The substrate 63 that has been selectively functionalized in the selected regions 61 of the surface 62 can now be provided with a cover 71, as is shown at the bottom in Figure 5 and in Figure 6. Said cover 71 forms together with the substrate 63 a microfluidic system 72 in which, in the example shown, two microfluidic channels 73 are provided, in each of which a selected region 61 has been functionalized.

If the channels 73 are now flushed with a mixture of protein 69 and Pluronic™, the functionalized regions 61 are activated by the protein 69, which is illustrated in the centre of Figure 6. The Pluronic deposits on the hydrophobic regions 68, as a result of which these regions are blocked in a cell-repelling manner.

If the channels 73 are now flushed with a solution containing biological cells 74, the cells 74 deposit only on the functionalized regions 61, which is illustrated at the bottom in Figure 6.

Before the closed microfluidic system 72 is flushed with the protein/Pluronic™ solution, the microfluidic system 72 closed in this way can be stored for many months without an appreciable decrease in the number of acid groups 67. In order to lengthen the storage life, the channels 73 can be filled with a fluid that prevents the acid groups 67 from coming into contact with alcohol molecules, which would lead to ester formation and thus impair the functionalization of the selected regions 61.

The microsystems 72 thus filled with a fluid, e.g. a noble gas or water, can then be stored for many months in a state in which they are packaged in a sterile fashion, and can be activated and subsequently colonized with biological cells 74 only on the part of the user.

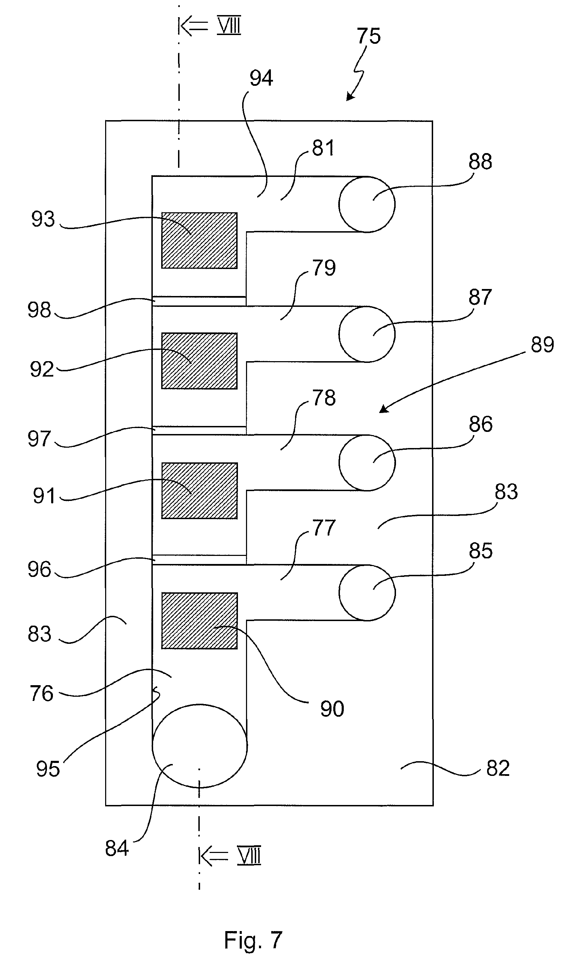

While Figures 1 to 4 showed a microfluidic system 12 in which only a selected region 47, 48 was provided for colonization with biological cells, Figures 7 to 9 show a further microfluidic system 75, in which a longitudinal channel 76 and four transverse channels 77, 78, 79 and 81 branching transversely therefrom are provided.

Figure 7 shows the novel microfluidic system 75 in plan view, wherein only a carrier plate 82 and wall regions 83 can be seen there, the cover plate having been removed.

The longitudinal channel 76 is provided with a connector 84 for microfluidic control, the transverse channels 77 to 81 being provided with connectors 85 to 88. A channel system 89 is formed in this way.

Functionalized regions 90, 91, 92 and 93 illustrated in hatched fashion are illustrated at the crossing point between the longitudinal channel 76 and the individual transverse channels 77 to 81. These functionalized regions 90 to 93 were hydrophilized in the manner described above in connection with Figure 5.

The remaining regions of the inner surface of the microfluidic system 75, that is to say that surface of the carrier plate 82 which is indicated at 94 and also that surface of the side walls of the channel structure 89 which is indicated at 95, were left hydrophobic, such that they can be activated in a cell-repelling manner.

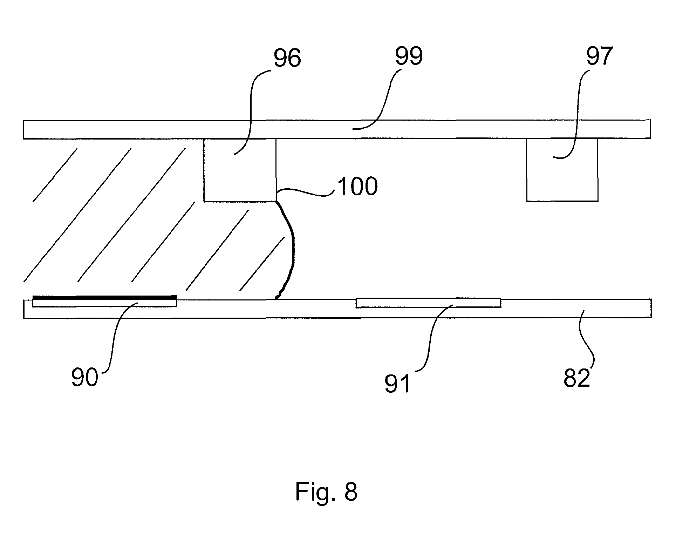

Three pressure barriers 96, 97 and 98 are also shown in the longitudinal channel 76, said pressure barriers leading to a cross-sectional alteration in the longitudinal channel 76, as is then shown in the longitudinal section in the form of a detail in Figure 8, viewed along the line VIII-VIII from Figure 7. Figure 8 shows firstly the carrier plate 82 and also a cover plate 99, which cannot be discerned in Figure 7 and on which the pressure barriers 96, 97 are arranged.

Two functionalized regions 90, 91 are illustrated on the carrier plate 82.

Figure 8 shows at the top that the pressure barrier 96 brings about a cross-sectional alteration behind the functionalized region 90, such that a fluid introduced from the left in Figure 8, that is to say via the connector 84 in Figure 7, tends to flow into the transverse channel 77 provided that a reduced pressure is generated at the outlet 85 there. This leads to the activation of the region 90.

The pressure barrier 96 can also be arranged on the carrier plate 82 or on other regions of the inner surface 94. What is important is that it provides for a surface tension effect by means of the cross-sectional constriction and the sharp edge 100 at the right-hand side of the pressure barrier 96 in Figure 8.

Provided that a reduced pressure is generated at the outlet 86, this has the effect together with the pressure barrier 97 that the fluid flows into the transverse channel 78.

The microfluidic control possible by means of the connectors 84 to 88 is thus supported by the pressure barriers 96 to 98.

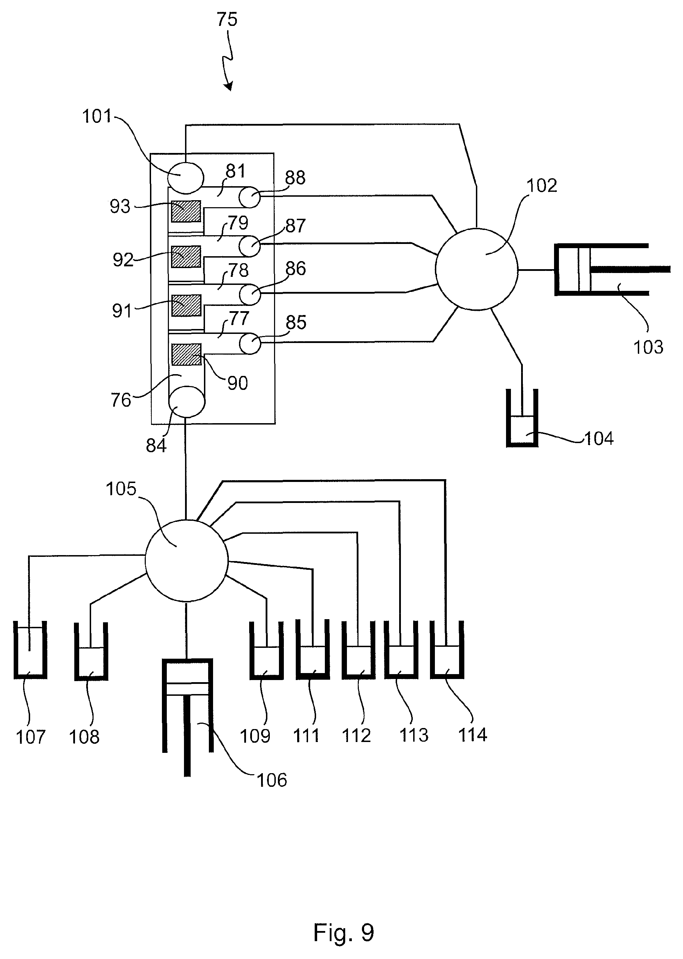

The way in which this can be used for selectively coating the functionalized regions 90 to 93 will now be explained with reference to Figure 9.

Figure 9 illustrates the microfluidic system 75 from Figure 7, the longitudinal channel 76 here being provided with a further connector 101.

The connector 101 of the longitudinal channel 76 and also the connectors 85 to 88 of the transverse channels 77 to 81 are connected to a valve 102, which is additionally connected to a piston pump 103 and a collecting vessel 104.

The connector 84 of the longitudinal channel 76 is connected to a valve 105, which is additionally connected to a piston pump 106 and seven supply vessels 107 to 114.

The valves 102, 105 can connect the assigned pumps 103 and 106, respectively, to one of the other connectors at the respective valve 102, 105.

The supply vessels 107 to 112 contain a washing solution in the supply vessel 108, an activation solution composed of a protein and Pluronic™ for activating the selected regions 90 to 93 in the supply vessel 107, and different cell suspensions in the supply vessels 109 to 113.

In a first step, the valve 105 connects the pump 106 to the washing solution in the supply vessel 108. The piston pump 106 is then filled with the washing solution.

The valve 105 then connects the piston pump 106 to the connector 84, while the valve 102 simultaneously connects the connector 101 to the pump 103. The pump 106 can then pump washing solution from the supply vessel 108 through the longitudinal channel 76, said washing solution being taken up by the pump 103. By means of corresponding control of the valve 102, the washing solution is then deposited into the collecting container 104.

Next, the piston pump 106 is then filled with the activation solution from the supply vessel 107.

The valve 105 then connects the piston pump 106 to the connector 84, while the piston pump 103 is connected to the connector 85 via the valve 102.

By means of actuation of the piston pumps 106 and 103, the activation solution is then conducted via the connector 84 and the lower segment of the longitudinal channel 76 into the first transverse channel 77. Since the connectors 86, 87, 88 and 101 are then closed, the activation solution - in a manner supported by the pressure barrier 96 - only comes into contact with the functionalized region 90.

In this way, the functionalized region 90 is activated by means of the protein from the reaction mixture, while at the same time the remaining surfaces in the channel system 89 which come into contact with the activation solution are passivated by the Pluronic™.

After the activation solution has been emptied from the piston pump 103 into the supply vessel 104, the piston pump 106 is then filled with the cell suspension from the supply vessel 109, whereupon in a corresponding manner the cell suspension is then guided exclusively over the now activated functionalized region 90, such that the latter is colonized with first cells.

In this case, no cells become established on the remaining regions of the surfaces, the described fluidic control and the pressure barrier 96 preventing cells from passing at all to the other functionalized regions 91 to 93, which, after all, have not yet been activated.

In the same way, the functionalized regions 91, 92 and 93 can firstly be activated with the reaction mixture from the supply container 107 and then be colonized with cells from the supply vessels 111 to 113.

The microfluidic system 75 can thus be colonized with different cells in an automated manner in order, for example, to establish a metabolic cascade with different cells. A reaction mixture is then conducted from the supply vessel 114 through the longitudinal channel 76 by means of the valves 102 and 105 and the piston pumps 103 and 106 in the manner described above, said reaction mixture being guided at a defined and variable flow rate successively to the different cell populations on the functionalized regions 90 to 93.

The cell populations then metabolize the substances contained in the reaction mixture and also the metabolites of cell populations situated upstream in the cascade. After passing through the entire metabolitic cascade, the reaction mixture emerges at the connector 101 and is temporarily stored in the collecting container 104 via the valve 102 and the piston pump 103. The substances and/or metabolites contained in this reaction mixture are then fed to an analysis.

In the microfluidic system 75, in a corresponding manner, instead of the extracellular proteins, different functional molecules such as enzymes or scavenger molecules can also be bound to the functionalized regions 90 to 93 in order to establish a "flowing through" system, for example. A reaction mixture is then conducted through the longitudinal channel 76, said reaction mixture being guided at a defined and variable flow rate successively to the different functional molecule populations on the functionalized regions 90 to 93.

If enzymes are used as functional molecules, the substrate molecules contained in the reaction mixture can then be converted in successive stages, if appropriate. After passing through the entire enzymatic reaction cascade, the reaction mixture is again temporarily stored in the collecting container 104 and then fed to an analysis. New and/or modified enzymes or enzymatically catalysed reaction sequences can be investigated and/or optimized in this way.

By contrast, if scavenger molecules such as antibodies or aptamers are used as functional molecules, then the microfluidic system 75 can also be used for diagnosis purposes by analysing the selective binding of ligands contained in the reaction mixture.

It goes without saying that it is also possible also to combine the three applications described with cells, enzymes and scavenger molecules, whereby complex biochemical reactions can be tested in vitro.

For establishing such a flow system with functional molecules instead of the ECM, in the microfluidic system 75 from Figure 9, activation solutions comprising the functional molecules and Pluronic™ are kept in store in the supply vessels 109 to 113 in order to successively and selectively activate the selected regions 90 to 93 in the manner described above and to passivate the other regions of the inner surface 94.

The supply vessel 114 now contains a reaction solution comprising the substrate molecules to be converted by the reaction cascade composed of different enzymes, or the ligands to be bound by means of the scavenger molecules.

The activation of the functionalized regions 90 to 93 and the implementation of the reaction cascade take place as follows in the case of enzymes as functional molecules:
The longitudinal channel 76 and also, if appropriate, the transverse channels 85 to 88 are firstly flushed in the manner described above.

Next, the piston pump 106 is then filled with an enzyme suspension from supply vessel 109. The valve 105 then connects the piston pump 106 to the connector 84, while the piston pump 103 is connected to the connector 85 via the valve 102.

By means of the actuation of the piston pumps 106 and 103, the enzyme suspension is then conducted into the first transverse channel 77 via the connector 84 and the lower segment of the longitudinal channel 76. Since the connectors 86, 87, 88 and 101 are then closed, the enzyme suspension - in a manner supported by the pressure barrier 76 - only comes into contact with the functionalized region 89.

In this way, the functionalized region 89 is activated by means of the enzyme from the enzyme suspension, while at the same time the remaining surfaces are passivated by the Pluronic™.

After the enzyme/Pluronic™ suspension has been emptied from the piston pump 103 into the supply vessel 104, the piston pump 106 is then filled with a further enzyme/Pluronic™ suspension from the supply vessel 111, whereupon the valve 102 is then set to the connector 86 in a corresponding manner in order to activate the next region 91 with the further enzyme. The regions already activated are already saturated, such that no further activation by the enzyme/Pluronic™ suspension flowing past from supply vessel 110 takes place there.

The same procedure is adopted with the remaining regions 92 and 93 to be activated.

After the activation process has been concluded, the pump 106 is emptied and washed and then filled with the reaction mixture from the supply vessel 114. Said reaction mixture, depending on the setting of the valve 102, can then be pumped via one or a plurality of regions activated with enzyme in the channel 76, where the molecules of the reaction mixture are chemically converted by the enzymes bound to the surface.

## Claims

1. Closed microfluidic system comprising a carrier plate (10, 63, 82) and a cover plate (14, 71, 99) as well as wall regions (15, 83, 25) arranged therebetween, which form a system (18, 89) of channels (16, 17, 76-81) and/or cavities with an inner surface, **characterized in that** selected regions (47, 48, 61, 90-93) of the inner surface are selectively functionalized by acid groups (67) formed in the functionalized regions (47, 48, 61, 90-93) on account of selective irradiation with short-wavelength light (66) such that the functionalized regions (47, 48, 61, 90-93) are hydrophilized.

2. Microfluidic system according to Claim 1, **characterized in that** the functionalized regions (47, 48, 61, 90-93) are hydrophilized by selective formation of acid groups (67) and the remaining regions of the inner surface are hydrophobic.

3. Microfluidic system according to Claim 1 or Claim 2, **characterized in that** at least one pressure barrier (96-98) is provided in the channels (16, 17, 76-81).

4. Microfluidic system according to anyone of Claims 1 to 3, **characterized in that** the carrier plate (10, 63, 82), the cover plate (14, 71, 99) and/or the wall regions (15, 83, 25) comprise a polymeric material.

5. Microfluidic system according to Claim 4, **characterized in that** the polymeric material is selected from the group comprising PDMS (polydimethylsiloxane), PMMA (poly(methyl methacrylate)), polystyrene, PEEK (polyether ether ketone), and COC (cyclic olefin copolymer).

6. Microfluidic system according to anyone of Claims 1 to 5, **characterized in that** an electrode arrangement (39, 40, 55) is provided in the channel system (18).

7. Method for producing a closed microfluidic system according to anyone of Claims 1 to 6, comprising the steps:
a) providing a carrier plate (10, 63, 82) and a cover plate (14, 71, 99), wherein wall regions (15, 83, 25) are provided on the carrier plate (10, 63, 82) and/or the cover plate (14, 71, 99),
b) selectively functionalizing selected regions (47, 48, 61, 90-93) of the inner surface on the carrier plate (10, 63, 82), the cover plate (14, 71, 99) and/or the wall regions (15, 83, 25), in that the selected regions (47, 48, 61, 90-93) are selectively irradiated with short-wavelength light (66) in order to form acid groups in the surface of the selected regions (47, 48, 61, 90-93), such that the selected regions (47, 48, 61, 90-93) are hydrophilized, and
c) permanently connecting carrier plate (10, 63, 82), cover plate (14, 71, 99) and wall regions (15, 83, 25) to form the closed microfluidic system (12, 72, 75).

8. Method according to Claim 7, **characterized in that**, in step b), the wavelength of the short-wavelength light (66) is in the range of 150 to 220 nm, preferably 180 to 200 nm, more preferably approximately 185 nm.

9. Method for the spatially resolved colonization of a closed microfluidic system (12, 72, 75) with biological cells (23, 24, 74), comprising the steps:
- providing the microfluidic system (12, 72, 75) according to anyone of Claims 1 to 6,
- flushing the microfluidic system (12, 72, 75) with at least one activation solution in order to activate the functionalized regions (47, 48, 61, 90-93) for the binding of the cells (23, 24, 74),
- flushing the microfluidic system (12, 72, 75) with a cell solution in order to bind the cells (23, 24, 74) to the activated regions (47, 48, 61, 90-93).

10. Method according to Claim 9, **characterized in that** the activation solution contains passivation molecules which adhere to the non-functionalized regions (47, 48, 61, 90-93) and lead to the passivation thereof.

11. Method according to Claim 9 or Claim 10, **characterized in that** the activation solution contains ligands which adhere to the functionalized regions (47, 48, 61, 90-93) and lead to the activation thereof.

12. Method according to Claim 11, **characterized in that** the ligands comprise protein molecules.

13. Method for establishing a closed microfluidic flow system in which substances contained in a reaction solution come into contact with differently activated regions (47, 48, 61, 90-93), comprising the steps:
- providing the microfluidic system (12, 72, 75) according to anyone of Claims 1 to 6, and
- flushing the microfluidic system (12, 72, 75) with at least one activation solution in order to activate the functionalized regions (47, 48, 61, 90-93) for a reaction with the substances.

14. Method according to Claim 13, **characterized in that** the activation solution contains passivation molecules which adhere to the non-functionalized regions (47, 48, 61, 90-93) and lead to the passivation thereof.

15. Method according to Claim 13 or Claim 14, **characterized in that** the activation solution contains functional molecules which adhere to the functionalized regions (47, 48, 61, 90-93) and lead to the activation thereof.

## Patentansprüche

1. Geschlossenes mikrofluidisches System mit einer Trägerplatte (10, 63, 82) und einer Deckelplatte (14, 71, 99) sowie dazwischen angeordneten Wandbereichen (15, 83, 25), die ein System (18, 89) von Kanälen (16, 17, 76-81) und/oder Kavitäten mit einer inneren Oberfläche bilden,
**dadurch gekennzeichnet, dass** ausgewählte Bereiche (47, 48, 61, 90-93) der inneren Oberfläche selektiv durch Säuregruppen (67) funktionalisiert sind, die in den funktionalisierten Bereichen (47, 48, 61, 90-93) infolge selektiver Bestrahlung mit kurzwelligem Licht (66) ausgebildete Säuregruppen (67) gebildet sind, so dass die funktionalisierten Bereiche (47, 48, 61, 90-93) hydrophilisiert sind.

2. Mikrofluidisches System nach Anspruch 1, **dadurch gekennzeichnet, dass** die funktionalisierten Bereiche (47, 48, 61, 90-93) durch selektive Ausbildung von Säuregruppen (67) hydrophilisiert und die übrigen Bereiche der inneren Oberflächen hydrophob sind.

3. Mikrofluidisches System nach Anspruch 1 oder Anspruch 2, **dadurch gekennzeichnet, dass** in den Kanälen (16, 17, 76-81) zumindest eine Druckbarriere (96-98) vorgesehen ist.

4. Mikrofluidisches System nach einem der Ansprüche 1 bis 3, **dadurch gekennzeichnet, dass** die Trägerplatte (10, 63, 82), die Deckelplatte (14, 71, 99) und/oder die Wandbereiche (15, 83, 25) ein polymeres Material aufweisen.

5. Mikrofluidisches System nach Anspruch 4, **dadurch gekennzeichnet, dass** das polymere Material ausgewählt ist aus der Gruppe, die PDMS (Polydimethylsiloxan), PMMA (Poly(methylmethacrylat)), Polystyrol, PEEK (Polyetheretherketon), und COC (cyclic olefin copolymer) umfasst.

6. Mikrofluidisches System nach einem der Ansprüche 1 bis 5, **dadurch gekennzeichnet, dass** in dem Kanalsystem (18) eine Elektrodenanordnung (39, 40, 55) vorgesehen ist.

7. Verfahren zur Herstellung eines geschlossenen mikrofluidischen Systems nach einem der Ansprüche 1 bis 6, mit den Schritten:
a) Bereitstellen einer Trägerplatte (10, 63, 82) und einer Deckelplatte(14, 71, 99), wobei an der Trägerplatte (10, 63, 82) und/oder der Deckelplatte (14, 71, 99) Wandbereiche (15, 83, 25) vorgesehen sind,
b) selektives Funktionalisieren von ausgewählten Bereichen (47, 48, 61, 90-93) der inneren Oberfläche an der Trägerplatte(10, 63, 82), der Deckelplatte (14, 71, 99) und/oder den Wandbereichen (15, 83, 25), indem die ausgewählten Bereiche (47, 48, 61, 90-93) selektiv mit kurzwelligem Licht (66) bestrahlt werden, um in der Oberfläche der ausgewählten Bereiche (47, 48, 61, 90-93) Säuregruppen auszubilden, so dass die ausgewählten Bereiche(47, 48, 61, 90-93) hydrophilisiert werden, und
c) dauerhaftes Verbinden von Trägerplatte(10, 63, 82), Deckelplatte(14, 71, 99) und Wandbereichen (15, 83, 25) zu dem geschlossenen mikrofluidischen System (12, 72, 75).

8. Verfahren nach Anspruch 7, **dadurch gekennzeichnet, dass** im Schritt b) die Wellenlänge des kurzwelligem Lichtes (66) im Bereich von 150 bis 220 nm, vorzugsweise bei 180 bis 200 nm, weiter vorzugsweise bei ca. 185 nm liegt.

9. Verfahren zur ortsaufgelösten Besiedelung eines mikrofluidischen Systems (12, 72, 75) mit biologischen Zellen (23, 24, 74), mit den Schritten:
- Bereitstellen des mikrofluidischen Systems (12, 72, 75) nach einem der Ansprüche 1 bis 6,
- Spülen des mikrofluidischen Systems (12, 72, 75) mit zumindest einer Aktivierungslösung, um die funktionalisierenden Bereiche (47, 48, 61, 90-93) für die Bindung der Zellen (23, 24, 74) zu aktivieren,
- Spülen des mikrofluidischen Systems (12, 72, 75) mit einer Zelllösung, um die Zellen (23, 24, 74) an die aktivierten Bereiche(47, 48, 61, 90-93) zu binden.

10. Verfahren nach Anspruch 9, **dadurch gekennzeichnet, dass** die Aktivierungslösung Passivierungsmoleküle enthält, die an den nicht funktionalisierten Bereichen(47, 48, 61, 90-93) anhaften und zu deren Passivierung führen.

11. Verfahren nach Anspruch 9 oder Anspruch 10, **dadurch gekennzeichnet, dass** die Aktivierungslösung Liganden enthält, die an den funktionalisierten Bereichen(47, 48, 61, 90-93) anhaften und zu deren Aktivierung führen.

12. Verfahren nach Anspruch 11, **dadurch gekennzeichnet, dass** die Liganden Proteinmoleküle umfassen.

13. Verfahren zur Etablierung eines mikrofluidischen Durchflusssystems, in dem in einer Reaktionslösung enthaltene Substanzen mit unterschiedlich aktivierten Bereichen (47, 48, 61, 90-93) in Kontakt gelangen, mit den Schritten:
- Bereitstellen des mikrofluidischen Systems(12, 72, 75) nach einem der Ansprüche 1 bis 6, und
- Spülen des mikrofluidischen Systems (12, 72, 75) mit zumindest einer Aktivierungslösung, um die funktionalisierenden Bereiche(47, 48, 61, 90-93) für eine Reaktion mit den Substanzen zu aktivieren.

14. Verfahren nach Anspruch 13, **dadurch gekennzeichnet, dass** die Aktivierungslösung Passivierungsmoleküle enthält, die an den nicht funktionalisierten Bereichen (47, 48, 61, 90-93) anhaften und zu deren Passivierung führen.

15. Verfahren nach Anspruch 13 oder Anspruch 14, **dadurch gekennzeichnet, dass** die Aktivierungslösung funktionale Moleküle enthält, die an den funktionalisierten Bereichen(47, 48, 61, 90-93) anhaften und zu deren Aktivierung führen.

## Revendications

1. Système microfluidique fermé comprenant une plaque de support (10, 63, 82) et une plaque de couverture (14, 71, 99) ainsi que des zones de paroi (15, 83, 25) agencées entre celles-ci, qui forment un système (18, 89) de canaux (16, 17, 76 à 81) et/ou de cavités avec une surface interne, **caractérisé en ce que** des zones sélectionnées (47, 48, 61, 90 à 93) de la surface interne sont sélectivement fonctionnalisées par des groupes acides (67) formés dans les zones fonctionnalisées (47, 48, 61, 90 à 93) en raison de l'irradiation sélective avec une lumière à courte longueur d'onde (66) de sorte que les zones fonctionnalisées (47, 48, 61, 90 à 93) soient rendues hydrophiles.

2. Système microfluidique selon la revendication 1, **caractérisé en ce que** les zones fonctionnalisées (47, 48, 61, 90 à 93) sont rendues hydrophiles par la formation sélective de groupes acides (67) et les zones restantes de la surface interne sont hydrophobes.

3. Système microfluidique selon la revendication 1 ou 2, **caractérisé en ce qu'**au moins une barrière de pression (96 à 98) est prévue dans les canaux (16, 17, 76 à 81).

4. Système microfluidique selon l'une quelconque des revendications 1 à 3, **caractérisé en ce que** la plaque de support (10, 63, 82), la plaque de couverture (14, 71, 99) et/ou les zones de paroi (15, 83, 25) comprennent un matériau polymère.

5. Système microfluidique selon la revendication 4, **caractérisé en ce que** le matériau polymère est choisi dans le groupe comprenant le PDMS (polydiméthylsiloxane), le PMMA (poly(méthacrylate de méthyle)), le polystyrène, la PEEK (polyétheréthercétone) et le COC (copolymère d'oléfine cyclique).

6. Système microfluidique selon l'une quelconque des revendications 1 à 5, **caractérisé en ce qu'**un agencement d'électrodes (39, 40, 55) est prévu dans le système de canaux (18).

7. Procédé pour fabriquer un système microfluidique fermé selon l'une quelconque des revendications 1 à 6, comprenant les étapes consistant :
a) à fournir une plaque de support (10, 63, 82) et une plaque de couverture (14, 71, 99), où des zones de paroi (15, 83, 25) sont prévues sur la plaque de support (10, 63, 82) et/ou la plaque de couverture (14, 71, 99),
b) à fonctionnaliser sélectivement des zones sélectionnées (47, 48, 61, 90 à 93) de la surface interne sur la plaque de support (10, 63, 82), la plaque de couverture (14, 71, 99) et/ou les zones de paroi (15, 83, 25), où les zones sélectionnées (47, 48, 61, 90 à 93) sont irradiées sélectivement avec une lumière à courte longueur d'onde (66) afin de former des groupes acides dans la surface des zones sélectionnées (47, 48, 61, 90 à 93), de sorte que les zones sélectionnées (47, 48, 61, 90 à 93) soient rendues hydrophiles, et
c) à relier de manière permanente la plaque de support (10, 63, 82), la plaque de couverture (14, 71, 99) et les zones de paroi (15, 83, 25) pour former le système microfluidique fermé (12, 72, 75).

8. Procédé selon la revendication 7, **caractérisé en ce que**, dans l'étape b), la longueur d'onde de la lumière à courte longueur d'onde (66) est dans la plage allant de 150 à 220 nm, de préférence de 180 à 200 nm, plus préférablement d'environ 185 nm.

9. Procédé pour la colonisation résolue spatialement d'un système microfluidique fermé (12, 72, 75) avec des cellules biologiques (23, 24, 74), comprenant les étapes consistant :
- à fournir le système microfluidique (12, 72, 75) selon l'une quelconque des revendications 1 à 6,
- à rincer le système microfluidique (12, 72, 75) avec au moins une solution d'activation afin d'activer les zones fonctionnalisées (47, 48, 61, 90 à 93) pour la liaison des cellules (23, 24, 74),
- à rincer le système microfluidique (12, 72, 75) avec une solution de cellules afin de lier les cellules (23, 24, 74) aux zones activées (47, 48, 61, 90 à 93).

10. Procédé selon la revendication 9, **caractérisé en ce que** la solution d'activation contient des molécules de passivation qui adhèrent aux zones non fonctionnalisées (47, 48, 61, 90 à 93) et conduisent à la passivation de celles-ci.

11. Procédé selon la revendication 9 ou 10, **caractérisé en ce que** la solution d'activation contient des ligands qui adhèrent aux zones fonctionnalisées (47, 48, 61, 90 à 93) et conduisent à l'activation de celles-ci.

12. Procédé selon la revendication 11, **caractérisé en ce que** les ligands comprennent des molécules de protéine.

13. Procédé pour établir un système d'écoulement microfluidique fermé dans lequel des substances contenues dans une solution de réaction entrent en contact avec des zones différemment activées (47, 48, 61, 90 à 93), comprenant les étapes consistant :
- à fournir le système microfluidique (12, 72, 75) selon l'une quelconque des revendications 1 à 6, et
- à rincer le système microfluidique (12, 72, 75) avec au moins une solution d'activation afin d'activer les zones fonctionnalisées (47, 48, 61, 90 à 93) pour une réaction avec les substances.

14. Procédé selon la revendication 13, **caractérisé en ce que** la solution d'activation contient des molécules de passivation qui adhèrent aux zones non fonctionnalisées (47, 48, 61, 90 à 93) et conduisent à la passivation de celles-ci.

15. Procédé selon la revendication 13 ou 14, **caractérisé en ce que** la solution d'activation contient des molécules fonctionnelles qui adhèrent aux zones fonctionnalisées (47, 48, 61, 90 à 93) et conduisent à l'activation de celles-ci.
